# EUROPEAN PATENT SPECIFICATION

(11) **EP 2 657 303 B1**
(45) Date of publication and mention of the grant of the patent: **29.07.2015**
(21) Application number: 11851511.3
(22) Date of filing: 10.11.2011
(51) Int. Cl.: C08L 101/00, C07D 471/04, C08K 5/3465, C09K 3/00

(54) **NEAR-INFRARED ABSORBING SYNTHETIC RESIN COMPOSITION**
NAHINFRAROT-ABSORBIERENDE KUNSTHARZZUSAMMENSETZUNG
COMPOSITION DE RÉSINE SYNTHÉTIQUE ABSORBANT DANS L'INFRAROUGE PROCHE

(30) Priority: 20.12.2010 JP 2010283286
(43) Date of publication of application: 30.10.2013
(73) Proprietor: Adeka Corporation, Tokyo 116-8554 (JP)
(72) Inventor: UEDA, Naoto, Saitama-shi Saitama 336-0022 (JP); NOMURA, Kazukiyo, Saitama-shi Saitama 336-0022 (JP); KAWAMOTO, Naoshi, Saitama-shi Saitama 336-0022 (JP)
(74) Representative: Forstmeyer, Dietmar
(86) International application number: PCT/JP2011/075944
(87) International publication number: WO 2012/086332

(56) References cited:
- JP-A- 1 114 801
- JP-A- 2001 194 522
- JP-A- 2002 122 731
- US-A- 3 751 419
- HIROYUKI HASHIMOTO ET AL. PREPARATION AND PROPERTIES OF DEHYDROTRICHOTOMINE-TYPE DYES: A NEW NEAR- INFRARED ABSOBING INDIGOID, HETEROCYCLES vol. 65, no. 6, 12 April 2005, pages 1385 - 1392

## Description

### TECHNICAL FIELD

The present invention relates to a near-infrared ray absorbing synthetic resin composition and a near-infrared ray absorbing material using the near-infrared ray absorbing synthetic resin composition. Specifically, the present invention relates to a near-infrared ray absorbing synthetic resin composition and a near-infrared ray absorbing material, which are useful for information recording materials utilizing laser light, which have absorption in the near-infrared region, various applications for which near-infrared ray absorbability (or heat ray absorbability) is required (for example, various optical filters for absorbing near-infrared ray, agriculture films, and exterior and interior materials for automobiles).

### BACKGROUND ART

Various applications of near-infrared ray absorbing materials (heat ray shielding materials) that absorb near-infrared ray have been suggested in recent years, and those having better performances are strongly demanded.
For example, materials such as methacrylic resins and polycarbonate resins have been used in so-called glazing applications including windows of buildings or vehicles such as automobiles, ceiling windows, doors or ceiling domes, and a material that can suppress the increase in room temperature while sufficiently taking visible light is desired.

Furthermore, greenhouses and vinyl greenhouses are actively used in the cultivation of plants so as to improve the yield amounts of agricultural crops or vary the harvesting periods, and thus a film that effectively shields against heat ray without substantially blocking the permeation of visible ray that is required for the growth of plants.

Furthermore, there are many cases in which near-infrared ray is used for driving or stopping electrical products that conduct the recording and playback of information recording media such as magnetic tapes, and it is necessary to block near-infrared ray from outside in such electrical products.

Furthermore, there is a problem that near-infrared ray that comes from a plasma display acts on surrounding electronic devices such as a codeless phone and a video cassette recorder for which a near-infrared ray remote controller is used, to thereby cause an improper operation. Therefore, a filter for plasma displays that exerts a near-infrared ray absorbing effect is desired.

Near-infrared ray absorbing pigments that absorb near-infrared ray have been conventionally used for near-infrared ray absorbing materials for use in these applications, and for example, cyanine-based pigments, phthalocyanine-based, polymethine-based pigments, squarylium-based pigments, porphyrin-based pigments, metal dithiol complex-based pigments, phthalocyanine-based pigments, diimonium-based pigments, inorganic oxide particles are used.

However, in the cases when these near-infrared ray absorbing pigments are used as near-infrared ray absorbing materials, these are used in combination with a synthetic resin such as a thermoplastic resin in many cases, and in many of such cases, the pigments had a problem in compatibility with the resin, and the pigments had an absorption wavelength in the visible ray region and thus impaired the transparency of the resin, and also impaired the physical property of the resin. Furthermore, the near-infrared ray absorbabilities of the pigments were not sufficient.

On the other hand, Non-patent Literature 1 describes a bipyrrolo isoquinoline compound, but this document does not describe at all a near-infrared ray absorbing synthetic resin composition and a near-infrared ray absorbing material which are used in combination with a resin, and the finding thereof cannot be obtained.

### CITATION LIST

### NON-PATENT LITERATURE

Non-patent Literature 1: HETEROCYCLES, Vol. 65, No. 6, 2005, pp. 1385-1392

### SUMMARY OF INVENTION

### TECHNICAL PROBLEM

Therefore, it is desirable to provide a near-infrared ray absorbing synthetic resin composition that is excellent in near-infrared ray absorbability and does not impair the original physical property of the resin, and to provide a near-infrared ray absorbing material that is excellent in near-infrared ray absorbability and has the original excellent physical property of the resin.

### SOLUTION TO PROBLEM

The present inventors intensively studied to solve the above-mentioned problem, and consequently found that the above-mentioned purpose can be achieved when a bipyrrolo isoquinoline compound is used, and completed the present invention.

Namely, the present invention provides a near-infrared ray absorbing synthetic resin composition containing a bipyrrolo isoquinoline compound represented by the following general formula (1) and a synthetic resin, and a near-infrared ray absorbing material formed by molding the near-infrared ray absorbing synthetic resin composition. 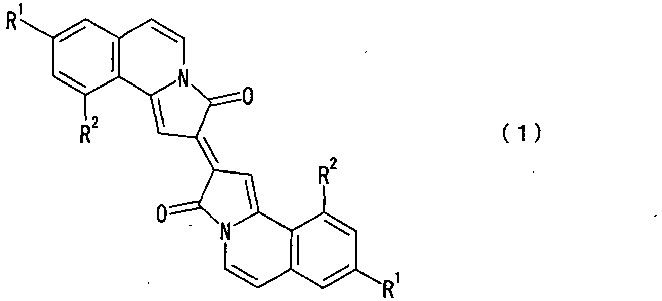 wherein R¹ and R² may be identical with or different from each other, and each represents a hydrogen atom, a halogen atom, an alkyl group having 1 to 20 carbon atoms optionally having substituents, an alkoxy group having 1 to 20 carbon atoms optionally having substituents, an aryl group having 6 to 20 carbon atoms optionally having substituents, an arylalkyl group having 7 to 20 carbon atoms optionally having substituents, or a cycloalkyl group having 5 to 12 carbon atoms optionally having substituents.

### ADVANTAGEOUS EFFECTS OF INVENTION

According to the present invention, a near-infrared ray absorbing synthetic resin composition that is excellent in near-infrared ray absorbability and does not impair the original physical property of the resin can be provided. Furthermore, when the near-infrared ray absorbing synthetic resin composition is molded, a near-infrared ray absorbing material that is excellent in near-infrared ray absorbability and also excellent in the original physical property of the resin can be provided.

### BRIEF DESCRIPTION OF DRAWINGS

Fig. 1 illustrates the absorption spectrum of compound No. 1 synthesized in Synthesis Example 1.
Fig. 2 illustrates the absorption spectrum of the polycarbonate near-infrared ray absorbing film obtained in Example 1.
Fig. 3 illustrates the absorption spectrum of the PMMA near-infrared ray absorbing film obtained in Example 2.

### DESCRIPTION OF EMBODIMENTS

Hereinafter the present invention will be described in detail based on the preferable exemplary embodiments.

First, the bipyrrolo isoquinoline compound used in the near-infrared ray absorbing synthetic resin composition of the present invention will be explained. The bipyrrolo isoquinoline compound is represented by the following general formula (1). 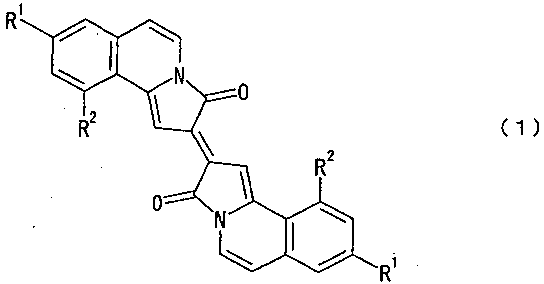 wherein R¹ and R² may be identical with or different from each other, and each represents a hydrogen atom, a halogen atom, an alkyl group having 1 to 20 carbon atoms optionally having substituents, an alkoxy group having 1 to 20 carbon atoms optionally having substituents, an aryl group having 6 to 20 carbon atoms optionally having substituents, an arylalkyl group having 7 to 20 carbon atoms optionally having substituents, or a cycloalkyl group having 5 to 12 carbon atoms optionally having substituents.

In the general formula (1), examples of the halogen atom represented by R¹ and R² may include fluorine, chlorine, bromine, iodine.

In the general formula (1), examples of the alkyl group having 1 to 20 carbon atoms optionally having substituents represented by R¹ and R² may include unsubstituted alkyl groups having 1 to 20 carbon atoms such as methyl, ethyl, n-propyl, isopropyl, n-butyl, isobutyl, sec-butyl, tert-butyl, n-pentyl, isopentyl, neopentyl, tert-amyl, 1,2-dimethylpropyl, n-hexyl, cyclohexyl, 1,3-dimethylbutyl, 1-isopropylpropyl, 1,2-dimethylbutyl, n-heptyl, 2-heptyl, 1,4-dimethylpentyl, tert-heptyl, 2-methyl-1-isopropylpropyl, 1-ethyl-3-methylbutyl, n-octyl, tert-octyl, 2-ethylhexyl, 2-methylhexyl, 2-propylhexyl, n-nonyl, isononyl, n-decyl, isodecyl, n-undecyl, isoundecyl, n-dodecyl, isododecyl, n-tridecyl, isotridecyl, n-tetradecyl, isotetradecyl, n-pentadecyl, isopentadecyl, n-hexadecyl, isohexadecyl, n-heptadecyl, isoheptadecyl, n-octadecyl, isooctadecyl, n-nonadecyl, isononadecyl, n-icosyl and isoicosyl.

Furthermore, in the general formula (1), examples of the alkoxy group having 1 to 20 carbon atoms optionally having substituents represented by R¹ and R² may include those corresponding to the above-mentioned alkyl groups, and specific examples may include methoxy, ethoxy, n-propoxy, isopropoxy, n-butoxy, isobutoxy, sec-butoxy, tert-butoxy, n-pentyloxy, isopentyloxy, neopentyloxy, 1,2-dimethyl-propoxy, n-hexyloxy, cyclohexyloxy, 1,3-dimethylbutoxy, 1-isopropylpropoxy.

Furthermore, in the general formula (1), examples of the aryl group having 6 to 20 carbon atoms optionally having substituents represented by R¹ and R² may include phenyl, naphthyl, anthracen-1-yl, phenanthren-1-yl.

Furthermore, in the general formula (1), examples of the arylalkyl group having 7 to 20 carbon atoms represented by R¹ and R² may include benzyl, phenethyl, 2-phenylpropan-2-yl, styryl, cinnamyl, diphenylmethyl, triphenylmethyl.

Furthermore, in the general formula (1), examples of the cycloalkyl group having 5 to 12 carbon atoms represented by R¹ and R² may include cyclopentyl, cyclohexyl, cyclooctyl, cyclododecyl, 4-methylcyclohexyl.

Examples of the substituents for the alkyl group having 1 to 20 carbon atoms optionally having substituents, the alkoxy group having 1 to 20 carbon atoms optionally having substituents, the aryl group having 6 to 20 carbon atoms optionally having substituents, the arylalkyl group having 7 to 20 carbon atoms optionally having substituents and the cycloalkyl group having 5 to 12 carbon atoms optionally having substituents, which are represented by R¹ and R², may include the following substituents.

Examples of the substituents may include alkyl groups such as methyl, ethyl, propyl, isopropyl, cyclopropyl, butyl, sec-butyl, tert-butyl, isobutyl, amyl, isoamyl, tert-amyl, cyclopentyl, hexyl, 2-hexyl, 3-hexyl, cyclohexyl, bicyclohexyl, 1-methylcyclohexyl, heptyl, 2-heptyl, 3-heptyl, isoheptyl, tert-heptyl, n-octyl, isooctyl, tert-octyl, 2-ethylhexyl, nonyl, isononyl and decyl; alkoxy groups such as methyloxy, ethyloxy, propyloxy, isopropyloxy, butyloxy, sec-butyloxy, tert-butyloxy, isobutyloxy, amyloxy, isoamyloxy, tert-amyloxy, hexyloxy, cyclohexyloxy, heptyloxy, isoheptyloxy, tert-heptyloxy, n-octyloxy, isooctyloxy, tert-octyloxy, 2-ethylhexyloxy, nonyloxy and decyloxy; alkylthio groups such as methylthio, ethylthio, propylthio, isopropylthio, butylthio, sec-butylthio, tert-butylthio, isobutylthio, amylthio, isoamylthio, tert-amylthio, hexylthio, cyclohexylthio, heptylthio, isoheptylthio, tert-heptylthio, n-octylthio, isooctylthio, tert-octylthio and 2-ethylhexylthio; alkenyl groups such as vinyl, 1-methylethenyl, 2-methylethenyl, 2-propenyl, 1-methyl-3-propenyl, 3-butenyl, 1-methyl-3-butenyl, isobutenyl, 3-pentenyl, 4-hexenyl, cyclohexenyl, bicyclohexenyl, heptenyl, octenyl, decenyl, pentadecenyl, eicosenyl and tricosenyl; arylalkyl groups such as benzyl, phenethyl, diphenylmethyl, triphenylmethyl, styryl and cinnamyl; aryl groups such as phenyl and naphthyl; aryloxy groups such as phenoxy and naphthyloxy; arylthio groups such as phenylthio and naphthylthio; heterocyclic groups such as pyridyl, pyrimidyl, pyridazyl, piperidyl, pyranyl, pyrazolyl, triazyl, pyrrolyl, quinolyl, isoquinolyl, imidazolyl, benzimidazolyl, triazolyl, furyl, furanyl, benzofuranyl, thienyl, thiophenyl, benzothiophenyl, thiadiazolyl, thiazolyl, benzothiazolyl, oxazolyl, benzoxazolyl, isothiazolyl, isoxazolyl, indolyl, 2-pyrrolidinon-1-yl, 2-piperidon-1-yl, 2,4-dioxyimidazolidin-3-yl and 2,4-dioxyoxazolidin-3-yl; halogen atoms such as fluorine, chlorine, bromine and iodine; acyl groups such as acetyl, 2-chloroacetyl, propionyl, octanoyl, acryloyl, methacryloyl, phenylcarbonyl (benzoyl), phthaloyl, 4-trifluoromethylbenzoyl, pivaloyl, salicyloyl, oxaloyl, stearoyl, methoxycarbonyl, ethoxycarbonyl, t-butoxycarbonyl, n-octadecyloxycarbonyl and carbamoyl; acyloxy groups such as acetyloxy and benzoyloxy; substituted amino groups such as amino, ethylamino, dimethylamino, diethylamino, butylamino, cyclopentylamino, 2-ethylhexylamino, dodecylamino, anilino, chlorophenylamino, toluidino, anisidino, N-methyl-anilino, diphenylamino, naphthylamino, 2-pyridylamino, methoxycarbonylamino, phenoxycarbonylamino, acetylamino, benzoylamino, formylamino, pivaloylamino, lauroylamino, carbamoylamino, N,N-dimethylaminocarbonylamino, N,N-diethylaminocarbonylamino, morpholinocarbonylamino, methoxycarbonylamino, ethoxycarbonylamino, t-butoxycarbonylamino, n-octadecyloxycarbonylamino, N-methyl-methoxycarbonylamino, phenoxycarbonylamino, sulfamoylamino, N,N-dimethylaminosulfonylamino, methylsulfonylamino, butylsulfonylamino and phenylsulfonylamino; a sulfonamide group, a sulfonyl group, a carboxyl group, a cyano group, a sulfo group, a hydroxyl group, a nitro group, a mercapto group, an imido group, a carbamoyl group, a sulfonamide group, and these groups may further be substituted. Furthermore, the carboxyl group and sulfo group may form a salt. In addition, in the cases when the alkyl group having 1 to 20 carbon atoms, the alkoxy group having 1 to 20 carbon atoms, the aryl group having 6 to 20 carbon atoms, the arylalkyl group having 7 to 20 carbon atoms and the cycloalkyl group having 5 to 12 carbon atoms each have substituents containing carbon atoms, the number of the carbon atoms including the carbon atoms included in the substituents is adjusted to be within each prescribed scope.

Furthermore, R¹ and R² are each preferably a hydrogen atom or an alkyl group having 1 to 20 carbon atoms optionally having substituents, more preferably a hydrogen atom or an unsubstituted alkyl group having 1 to 4 carbon atoms, even more preferably a hydrogen atom or a methyl group, from the viewpoint of near-infrared ray absorbability.
In addition, the two R¹s in the general formula (1) may be identical with or different from each other, and this is also the case with R².

Specific examples of the bipyrrolo isoquinoline compound represented by the general formula (1), which is used in the near-infrared ray absorbing synthetic resin composition of the present invention, may include the following compounds No. 1 to No. 12, but are not limited to these compounds.

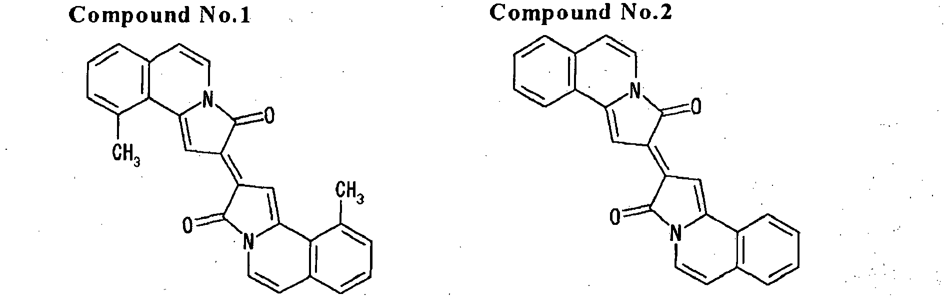

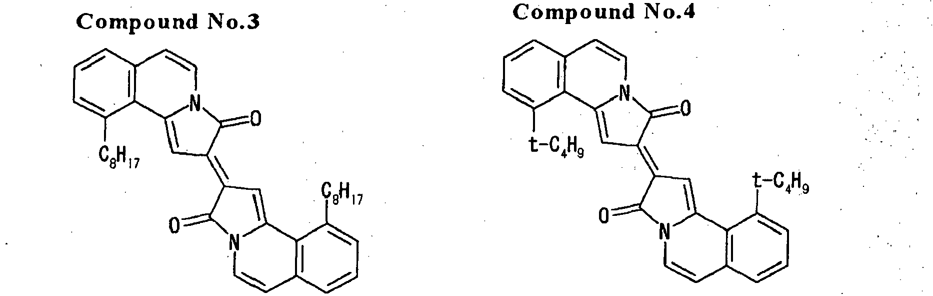

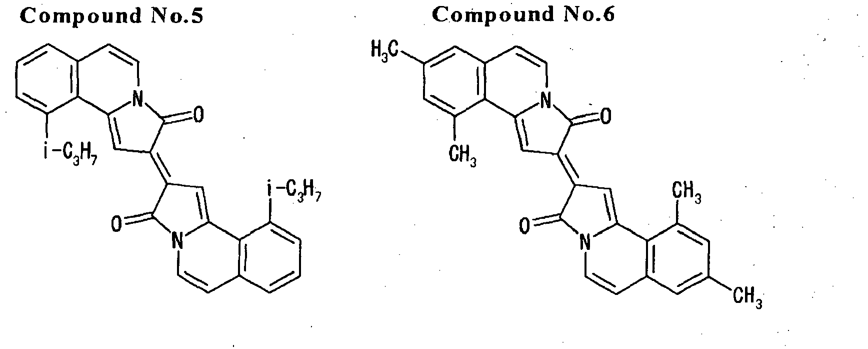

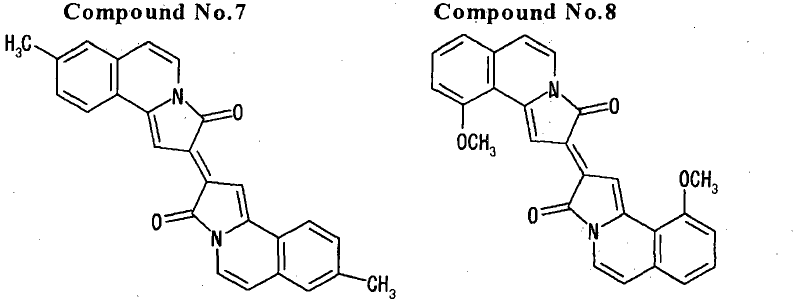

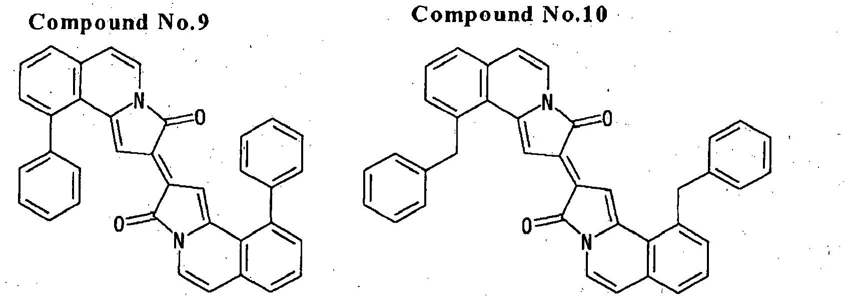

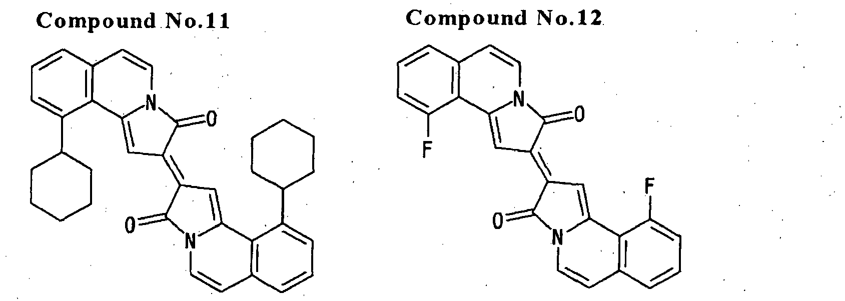

The bipyrrolo isoquinoline compound represented by the above-mentioned general formula (1) can be produced by applying a conventionally-known reaction. For example, the bipyrrolo isoquinoline compound represented by the above-mentioned general formula (1) can be obtained by reacting a compound represented by the following general formula (2), which is synthesized from a 4-(substituted phenyl)-4-oxo-2-butenoic acid, with 2-aminoacetaldehyde dimethylacetal to give a compound represented by the following general formula (3), and treating the obtained compound represented by the general formula (3) with aluminum chloride. 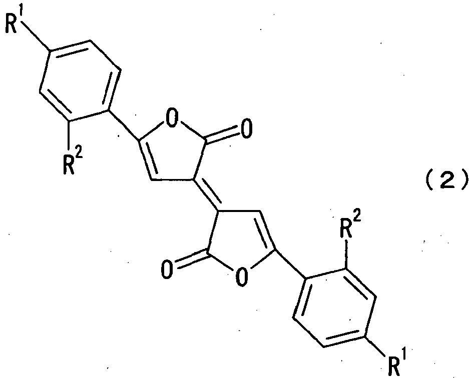 wherein R¹ and R² are the same as in the above general formula (1). 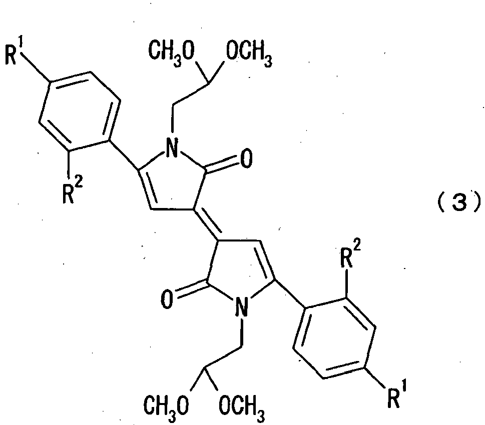 wherein R¹ and R² are the same as in the above general formula (1).

Next, the synthetic resin that is used in the near-infrared ray absorbing synthetic resin composition of the present invention will be explained.

Examples of the synthetic resin that can be used in the present invention may include thermoplastic resins, thermosetting resins, fluorine-based resins, silicone resins.

Examples of the thermoplastic resins may include thermoplastic resins such as α-olefin polymers such as polypropylene, high density polyethylene, low density polyethylene, straight chain low density polyethylene, crosslinked polyethylene, ultrahigh molecular weight polyethylene, polybutene-1, poly-3-methylpentene and poly-4-methylpentene or polyolefin-based resins such as ethylene-vinyl acetate copolymer, ethylene-ethyl acrylate copolymer and ethylene-propylene copolymer, and copolymers thereof; halogen-containing resins such as polyvinyl chloride, polyvinylidene chloride, polyethylene chloride, polypropylene chloride, polyvinylidene fluoride, chlorinated rubber, vinyl chloride-vinyl acetate copolymer, vinyl chloride-ethylene copolymer, vinyl chloride-vinylidene chloride copolymer, vinyl chloride-vinylidene chloride-vinyl acetate terpolymer, vinyl chloride-acrylic acid ester copolymer, vinyl chloride-maleic acid ester copolymer and vinyl chloride-cyclohexylmaleimide copolymer; petroleum resins, coumarone resins, polystyrenes, polyvinyl acetate, acrylic resins, copolymers of styrene and/or α-methylstyrene with other monomers (for example, maleic anhydride, phenylmaleimide, methyl methacrylate, butadiene, acrylonitrile) (for example, AS resin, ABS resin, ACS resin, SBS resin, MBS resin, heat-resistant ABS resin); polymethyl methacrylate, polyvinyl alcohol, polyvinyl formal, polyvinyl butyral; polyalkylene telephthalates such as polyethylene telephthalate, polybutylene telephthalate and polycyclohexanedimethylene telephthalate, aromatic polyesters such as polyalkylene naphthalates such as polyethylene naphthalate and polybutylene naphthalate, and straight chain polyesters such as polytetramethylene telephthalate; degradable aliphatic polyesters such as polyhydroxy butyrate, polycaprolactone, polybutylene succinate, polyethylene succinate, polylactic acid, polymalic acid, polyglycolic acid, polydioxane and poly(2-oxetanone); polyphenylene oxide, polyamides such as polycaprolactam and polyhexamethylene adipamide, polycarbonates, polycarbonate/ABS resins, branched polycarbonates, polyacetal, polyphenylene sulfide, polyurethanes, cellulose-based resins and polyimide resins, and blends thereof.

Furthermore, the thermoplastic resins may be elastomers such as isoprene rubbers, butadiene rubbers, acrylonitrile-butadiene copolymerized rubbers, styrene-butadiene copolymerized rubbers, fluorine rubbers, silicone rubbers, olefin-based elastomers, styrene-based elastomers, polyester-based elastomers, nitrile-based elastomers, nylon-based elastomers, vinyl chloride-based elastomers, polyamide-based elastomers and polyurethane-based elastomers.

Examples of the thermosetting resins may include phenolic resins, urea resins, melamine resins, epoxy resins, unsaturated polyester resins.

Furthermore, examples of the synthetic resins may include silicone rubber polyethersulfones, polysulfones, polyphenylene ethers, polyether ketones, poly ether ether ketones, liquid crystal polymers.

In the present invention, these synthetic resins may be used alone, or two or more kinds may be used. Furthermore, the synthetic resins may be in the form of alloy. These synthetic resins can be used irrespective of the molecular weight, the polymerization degree, the density, the softening point, the ratio of insoluble components in a solvent, the degree of steric regularity, the presence or absence of a catalyst residue, the kinds and incorporation ratio of monomers as raw materials, the kind of a polymerization catalyst (for example, a Ziegler catalyst, a metallocene catalyst .

Among the synthetic resins, thermoplastic resins are preferable from the viewpoints of the compatibility and processability of the bipyrrolo isoquinoline compound represented by the above-mentioned general formula (1), and among the thermoplastic resins, polycarbonates and polymethyl methacrylate are specifically preferable from the viewpoints of transparency and near-infrared ray absorbability.

In the near-infrared ray absorbing synthetic resin composition of the present invention, the content of the bipyrrolo isoquinoline compound of the above-mentioned the general formula (1) is 0.001 to 5 parts by mass, with respect to 100 parts by mass of the above-mentioned synthetic resin.

When the content of the bipyrrolo isoquinoline compound of the general formula (1) is lower than 0.001 part by mass, it is possible that sufficient near-infrared ray absorbability cannot be achieved, whereas when the content goes beyond 5 parts by mass, it is not cost efficient since an effect that is worth the use amount cannot be obtained, and it is also possible that the transparency in the visible region is impaired.

The method for incorporating the bipyrrolo isoquinoline compound represented by the general formula (1) into the synthetic resin may follow a conventional method, and is not specifically limited. For example, in the case when a thermoplastic resin is used as the synthetic resin, any method that is generally used in the cases when various additives are added to a thermoplastic resin can be used, and for example, the thermoplastic resin may be incorporated by mixing and kneading by roll kneading, bumper kneading, an extruder, a kneader.

Alternatively, a solution of the near-infrared ray absorbing synthetic resin composition obtained by dissolving or dispersing the above-mentioned bipyrrolo isoquinoline compound represented by the general formula (1) and the above-mentioned synthetic resin in various solvents may be incorporated and used.

Where necessary, additives used in synthetic resins such as phenol-based antioxidants, phosphorus-based antioxidants, thioether-based antioxidants, ultraviolet absorbers and hindered amine-based light stabilizers may be incorporated and stabilized in the near-infrared ray absorbing synthetic resin composition of the present invention.

Examples of the above-mentioned phenol-based antioxidants may include 2,6-di-tert-butyl-p-cresol, 2,6-diphenyl-4-octadecyloxyphenol, distearyl (3,5-di-tert-butyl-4-hydroxybenzyl)phosphonate, 1,6-hexamethylenebis[(3,5-di-tert-butyl-4-hydroxyphenyl)propionic acid amide], 4,4'-thiobis(6-tert-butyl-m-cresol), 2,2'-methylenebis(4-methyl-6-tert-butylphenol), 2,2'-methylenebis(4-ethyl-6-tert-butylphenol), 4,4'-butylidenebis(6-tert-butyl-m-cresol), 2,2'-ethylidenebis(4,6-di-tert-butylphenol), 2,2'-ethylidenebis(4-sec-butyl-6-tert-butylphenol), 1,1,3-tris(2-methyl-4-hydroxy-5-tert-butylphenyl)butane, 1,3,5-tris(2,6-dimethyl-3-hydroxy-4-tert-butylbenzyl)isocyanurate, 1,3,5-tris(3,5-di-tert-butyl-4-hydroxybenzyl)isocyanurate, 1,3,5-tris(3,5-di-tert-butyl-4-hydroxybenzyl)-2,4,6-trimethylbenzene, 2-tert-butyl-4-methyl-6-(2-acryloyloxy-3-tert-butyl-5-methylbenzyl)phenol, stearyl (3,5-di-tert-butyl-4-hydroxyphenyl)propionate, tetrakis[methyl 3-(3,5-di-tert-butyl-4-hydroxyphenyl)propionate]methane, thiodiethylene glycol bis[(3,5-di-tert-butyl-4-hydroxyphenyl)propionate], 1,6-hexamethylenebis[(3,5-di-tert-butyl-4-hydroxyphenyl)propionate], bis[3,3-bis(4-hydroxy-3-tert-butylphenyl)butylic acid] glycol ester, bis[2-tert-butyl-4-methyl-6-(2-hydroxy-3-tert-butyl-5-methylbenzyl)phenyl] telephthalate, 1,3,5-tris[(3,5-di-tert-butyl-4-hydroxyphenyl)propionyloxyethyl] isocyanurate, 3,9-bis[1,1-dimethyl-2-{(3-tert-butyl-4-hydroxy-5-methylphenyl)propionyloxy}ethyl]-2 ,4,8,10-tetraoxaspiro[5,5]undecane, triethylene glycol bis[(3-tert-butyl-4-hydroxy-5-methylphenyl)propionate], and they are used by preferably 0.001 to 10 parts by mass, more preferably 0.05 to 5 parts by mass with respect to 100 parts by mass of the synthetic resin.

Examples of the above-mentioned phosphorus-based antioxidant may include trisnonylphenyl phosphite, tris[2-tert-butyl-4-(3-tert-butyl-4-hydroxy-5-methylphenylthio)-5-methylphenyl] phosphite, tridecyl phosphite, octyl diphenyl phosphite, di(decyl) monophenyl phosphite, di(tridecyl) pentaerythritol diphosphite, di(nonylphenyl) pentaerythritol diphosphite, bis(2,4-di-tert-butylphenyl) pentaerythritol diphosphite, bis(2,6-di-tert-butyl-4-methylphenyl) pentaerythritol diphosphite, bis(2,4,6-tri-tert-butylphenyl) pentaerythritol diphosphite, bis(2,4-dicumylphenyl) pentaerythritol diphosphite, tetra(tridecyl) isopropylidenediphenol diphosphite, tetra(tridecyl)-4,4'-n-butylidenebis(2-tert-butyl-5-methylphenol) diphosphite, hexa(tridecyl)-1,1,3-tris(2-methyl-4-hydroxy-5-tert-butylphenyl)butanetriphosphite, tetrakis(2,4-di-tert-butylphenyl)biphenylenediphosphonite, 9,10-dihydro-9-oxa-10-phosphaphenanthrene-10-oxide, 2,2'-methylenebis(4,6-tert-butylphenyl)-2-ethylhexyl phosphite, 2,2'-methylenebis(4,6-tert-butylphenyl)-octadecyl phosphite, 2,2'-ethylidenebis(4,6-di-tert-butylphenyl)fluorophosphite, tris(2-[(2,4,8,10-tetrakistert-butyldibenzo[d,f][1,3,2]dioxaphosphepin-6-yl)oxy]ethyl)a mine, phosphites of 2-ethyl-2-butylpropylene glycol and 2,4,6-tri-tert-butylphenol, and they are used by preferably 0.001 to 10 parts by mass, more preferably 0.05 to 5 parts by mass, with respect to 100 parts by mass of the synthetic resin.

Examples of the above-mentioned thioether-based antioxidants may include dialkyl thiodipropionates such as dilauryl thiodipropionate, dimyristyl thiodipropionate and distearyl thiodipropionate, and pentaerythritol tetrakis(β-alkylthiopropionic acid esters), and they are used by preferably 0.001 to 10 parts by mass, more preferably 0.05 to 5 parts by mass, with respect to 100 parts by mass of the synthetic resin.

Examples of the above-mentioned ultraviolet absorber may include 2-hydroxybenzophenones such as 2,4-dihydroxybenzophenone, 2-hydroxy-4-methoxybenzophenone, 2-hydroxy-4-octoxybenzophenone and 5,5'-methylenebis(2-hydroxy-4-methoxybenzophenone); 2-(2'-hydroxyphenyl)benzotriazoles such as 2-(2'-hydroxy-5'-methylphenyl)benzotriazole, 2-(2'-hydroxy-3',5'-di-tert-butylphenyl)-5-chlorobenzotriazole, 2-(2'-hydroxy-3'-tert-butyl-5'-methylphenyl)-5-chlorobenzotriazole, 2-(2'-hydroxy-5'-tert-octylphenyl)benzotriazole, 2-(2'-hydroxy-3',5'-dicumylphenyl)benzotriazole, 2,2'-methylenebis(4-tert-octyl-6-(benzotriazolyl)phenol) and 2-(2'-hydroxy-3'-tert-butyl-5'-carboxyphenyl)benzotriazole; benzoates such as phenyl salicylate, resorcinol monobenzoate, 2,4-di-tert-butylphenyl-3,5-di-tert-butyl-4-hydroxybenzoate, 2,4-di-tert-amylphenyl-3,5-di-tert-butyl-4-hydroxybenzoate and hexadecyl-3,5-di-tert-butyl-4-hydroxybenzoate; substituted oxanilides such as 2-ethyl-2'-ethoxyoxanilide and 2-ethoxy-4'-dodecyloxanilide; cyanoacrylates such as ethyl-α-cyano-β, β-diphenylacrylate and methyl-2-cyano-3-methyl-3-(p-methoxyphenyl)acrylate; triaryltriazines such as 2-(2-hydroxy-4-octoxyphenyl)-4,6-bis(2,4-di-tert-butylphenyl)-s-triazine, 2-(2-hydroxy-4-methoxyphenyl)-4,6-diphenyl-s-triazine and 2-(2-hydroxy-4-propoxy-5-methylphenyl)-4,6-bis(2,4-di-tert-butylphenyl)-s-triazine, and they are used by preferably 0.001 to 30 parts by mass, more preferably 0.05 to 10 parts by mass with respect to 100 parts by mass of the synthetic resin.

Examples of the above-mentioned hindered amine-based light stabilizers may include hindered amine compounds such as 2,2,6,6-tetramethyl-4-piperidyl stearate, 1,2,2,6,6-pentamethyl-4-piperidyl stearate, 2,2,6,6-tetramethyl-4-piperidyl benzoate, bis(2,2,6,6-tetramethyl-4-piperidyl) sebacate, bis(1,2,2,6,6-tetramethyl-4-piperidyl) sebacate, bis(1-octoxy-2,2,6,6-tetramethyl-4-piperidyl) sebacate, tetrakis(2,2,6,6-tetramethyl-4-piperidyl)-1,2,3,4-butanetetracarboxylate, tetrakis(1,2,2,6,6-pentamethyl-4-piperidyl)-1,2,3,4-butanetetracarboxylate, bis(2,2,6,6-tetramethyl-4-piperidyl)-di(tridecyl)-1,2,3,4-butanetetracarboxylate, bis(1,2,2,6,6-pentamethyl-4-piperidyl)-di(tridecyl)-1,2,3,4-butanetetracarboxylate, bis(1,2,2,4,4-pentamethyl-4-piperidyl)-2-butyl-2-(3,5-di-tert-butyl-4-hydroxybenzyl)ma lonate, 1-(2-hydroxyethyl)-2,2,6,6-tetramethyl-4-piperidinol/diethyl succinate polycondensate, 1,6-bis(2,2,6,6-tetramethyl-4-piperidylamino)hexane/2,4-dichloro-6-morpholino-s-triazi ne polycondensate, 1,6-bis(2,2,6,6-tetramethyl-4-piperidylamino)hexane/2,4-dichloro-6-tert-octylamino-s-tr iazine polycondensate, 1,5,8,12-tetrakis[2,4-bis(N-butyl-N-(2,2,6,6-tetramethyl-4-piperidyl)amino)-s-triazin-6-yl]-1,5,8,12-tetraazadodecane, 1,5,8,12-tetrakis[2,4-bis(N-butyl-N-(1,2,2,6,6-pentamethyl-4-piperidyl)amino)-s-triazin -6-yl]-1,5,8-12-tetraazadodecane, 1,6,11-tris[2,4-bis(N-butyl-N-(2,2,6,6-tetramethyl-4-piperidyl)amino)-s-triazin-6-yl]ami noundecane and 1,6,11-tris[2,4-bis(N-butyl-N-(1,2,2,6,6-pentamethyl-4-piperidyl)amino)-s-triazin-6-yl]a minoundecane, and they are used by preferably 0.001 to 30 parts by mass, more preferably 0.05 to 10 parts by mass with respect to 100 parts by mass of the synthetic resin.

Furthermore, where necessary, additives that are generally incorporated in synthetic resins such as nucleating agents such as aluminum p-tert-butylbenzoate, metal salts of aromatic phosphate esters and dibenzylidene sorbitol, antistatic agents, metal soaps, hydrotalcites, triazine ring-containing compounds, metal hydroxides, phosphate ester-based flame retardants, condensed phosphate ester-based flame retardants, phosphate-based flame retardants, inorganic phosphorus-based flame retardants, (poly)phosphate salt-based flame retardants, halogen-based flame retardants, silicon-based flame retardants, antimony oxides such as antimony trioxide, other inorganic-based flame retardant aids, other organic-based flame retardant aids, fillers, pigments, lubricants and foaming agents may be added to the near-infrared ray absorbing synthetic resin composition of the present invention.

Examples of the above-mentioned triazine ring-containing compounds may include melamine, ammeline, benzoguanamine, acetoguanamine, phthalodiguanamine, melamine cyanurate, melamine pyrophosphate, butylene diguanamine, norbornene diguanamine, methylene diguanamine, ethylene dimelamine, trimethylene dimelamine, tetramethylene dimelamine, hexamethylene dimelamine, 1,3-hexylenedimelamine.

Examples of the above-mentioned metal hydroxides may include magnesium hydroxide, aluminum hydroxide, calcium hydroxide, barium hydroxide, zinc hydroxide, KISUMA 5A (magnesium hydroxide: manufactured by Kyowa Chemical industry Co., Ltd.).

Examples of the above-mentioned phosphate ester-based flame retardants may include trimethyl phosphate, triethyl phosphate, tributyl phosphate, tributoxyethyl phosphate, trischloroethyl phosphate, trisdichloropropyl phosphate, triphenyl phosphate, tricresyl phosphate, cresyl diphenyl phosphate, trixylenyl phosphate, octyl diphenyl phosphate, xylenyl diphenyl phosphate, trisisopropyl phenyl phosphate, 2-ethylhexyl diphenyl phosphate, t-butylphenyl diphenyl phosphate, bis-(t-butylphenyl) phenyl phosphate, tris-(t-butylphenyl) phosphate, isopropyl phenyl diphenyl phosphate, bis-(isopropylphenyl) diphenyl phosphate, tris-(isopropylphenyl) phosphate.

Examples of the above-mentioned condensed phosphate ester-based flame retardants may include 1,3-phenylenebis(diphenyl phosphate), 1,3-phenylenebis(dixylenyl phosphate), bisphenol A bis(diphenyl phosphate).

Examples of the above-mentioned (poly)phosphate salt-based flame retardants may include ammonium salts and amine salts of (poly)phosphoric acids such as ammonium polyphosphate, melamine polyphosphate, piperazine polyphosphate, melamine pyrophosphate and piperazine pyrophosphate.

Examples of the above-mentioned other inorganic-based flame retardant aids may include inorganic compounds such as titanium oxide, aluminum oxide, magnesium oxide, hydrotalcites, talc and montmollironite, and surface-treated products thereof, and for example, various commercially available products such as TIPAQUER-680 (titanium oxide: manufactured by Ishihara Sangyo Kaisha, Ltd.), KYOWAMAG 150 (magnesium oxide: manufactured by Kyowa Chemical industry Co., Ltd.), DHT-4A (hydrotalcite: manufactured by Kyowa Chemical industry Co., Ltd.) and ALCAMIZER 4 (zinc-modified hydrotalcite: manufactured by Kyowa Chemical industry Co., Ltd.) can be used.
Examples of the above-mentioned other organic-based flame retardant aids may include pentaerythritol.

Furthermore, where necessary, additives that are generally used in synthetic resins such as crosslinking agents, antifogging agents, plate-out preventing agents, surface treating agents, plasticizers, lubricants, flame retardants, antifogging agents, fluorescent agents, anti-mold agents, bactericides, foaming agents, metal inactivators, mold release agents, pigments, processing aids, antioxidants, light stabilizers can be incorporated in the near-infrared ray absorbing synthetic resin composition of the present invention to the extent that the effect of the present invention is not impaired.

In the case when the bipyrrolo isoquinoline compound represented by the above-mentioned general formula (1) and optional additives other than the above-mentioned synthetic resin are incorporated in the near-infrared ray absorbing synthetic resin composition of the present invention, although the incorporation amount thereof can be suitably selected depending on the kinds of the additives, it is preferable that the incorporation amount is adjusted to 20 parts by mass or less in total with respect to 100 parts by mass of the synthetic resin from the viewpoint of avoidance of the impairment of the effect of the present invention.

The near-infrared ray absorbing synthetic resin composition of the present invention can be formed into a molded article as a near-infrared ray absorbing material by molding. The molding method is not specifically limited, and extrusion processing, calendar processing, injection molding, rolling, compression molding, blow molding may be exemplified, and molded products having various shapes such as resin plates, sheets, films, fibers and profiles can be produced.

Alternatively, near-infrared ray absorbing films can be formed as near-infrared ray absorbing materials by dissolving the near-infrared ray absorbing synthetic resin composition of the present invention in various solvents and preparing cast films.

The near-infrared ray absorbing material obtained by the near-infrared ray absorbing synthetic resin composition of the present invention is excellent in near-infrared ray absorbability.

The near-infrared ray absorbing synthetic resin composition and near-infrared ray absorbing material of the present invention can be used in various applications for which near-infrared ray absorbability (heat ray absorbability) is required, including optical information recording materials such as optical cards, organic photoconductors, laser heat transfer recording materials, laser thermosensitive recording materials and materials for laser direct plate making; various optical filters for absorbing near-infrared ray such as filters for plasma displays, optical filters for thin displays and optical filters for photosemiconductor devices; heat ray shielding materials, heat ray shielding films and heat ray shielding resin glasses; thermal insulation-thermal storage fibers; protective eyeglasses, agricultural films, interior and exterior materials for automobiles, sheets and other various resin-molded articles; secret inks, coating materials.

### EXAMPLES

Hereinafter the present invention will further be explained in more detail with referring to Examples. In the following Examples, unless otherwise indicated, % and ppm are based on mass.

### [Synthesis Example 1] Synthesis of compound No. 1

344 mg (1.00 mmol) of the following compound A, 0.48 g (4.6 mmol) of 2-aminoacetaldehyde dimethylacetal and 4 ml of dichloromethane were stirred overnight in a three-necked flask under a nitrogen atmosphere at room temperature. Thereafter, 10 ml of 10% hydrochloric acid was added to the reaction liquid. The mixed liquid was stirred overnight and extracted with chloroform. The extracted chloroform solution was dried over anhydrous sodium sulfate and concentrated under a reduced pressure. The concentrate was purified by column chromatography to give 176 mg (yield 34%) of the following compound B.

103 mg (0.20 mmol) of the obtained compound B, 0.50 g (3.8 mmol) of anhydrous aluminum chloride and 10 ml of dichloromethane were stirred overnight in a three-necked flask under a nitrogen atmosphere at room temperature. Thereafter, 10% hydrochloric acid and chloroform were then added to the reaction liquid under stirring. The precipitated solid substance was separated by filtration to give 49 mg (yield 63%) of a powder.

The obtained powder was analyzed by FT-IR measurement and mass spectroscopy. The results of the analyses are illustrated below. By the following results of the analyses, the obtained powder was identified as compound No. 1 (the structural formula is as mentioned above).

### <Results of analyses>

- Result of FT-IR measurement (cm⁻¹)
   1677, 1630, 1510
- Result of mass spectroscopy (EI-MS: jms-K9 manufactured by JEOL, Ltd.)
   M=390

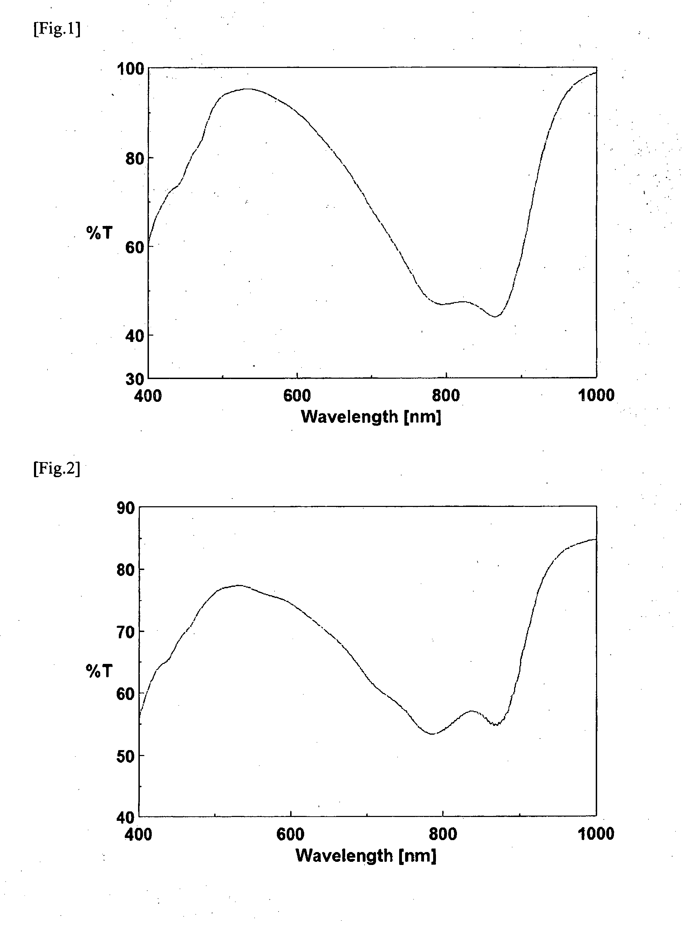

Furthermore, the absorption spectrum of a chloroform solution (concentration 0.001%) of the obtained compound No. 1 was measured. The measurement was conducted by V-670 manufactured by JASCO Corporation. The absorption spectrum is illustrated in Fig. 1. The maximum absorb wavelength was 864 nm, and the molar absorption coefficient ε = 1.4 × 10⁴.

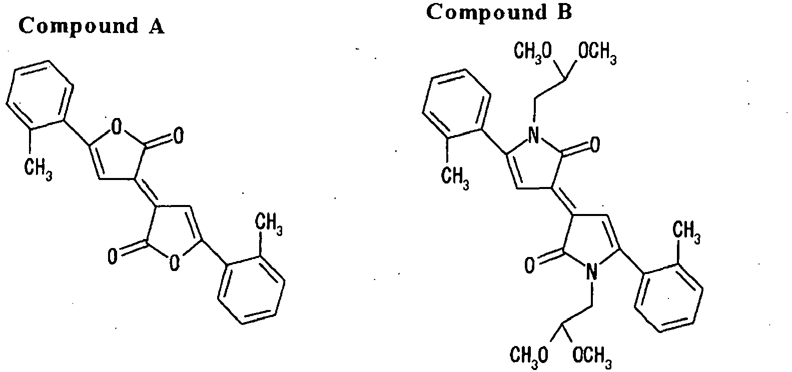

### [Example 1] Production of near-infrared ray absorbing synthetic resin composition and near-infrared ray absorbing material

1.25 g of polycarbonate (EUPILON S-3000F (manufactured by Mitsubishi Engineering-Plastics Corporation)), 3.75 mg of compound No. 1 obtained in Synthesis Example 1 and dichloromethane were put into a 100 ml measurement flask, sufficiently dissolved, and diluted in a measuring cylinder to 100 ml to thereby give a solution of the near-infrared ray absorbing synthetic resin composition. 10 ml of this solution was taken into a petri dish and slowly dried to give a polycarbonate near-infrared ray absorbing film having a thickness of 25 µm.

The absorption spectrum of the obtained polycarbonate near-infrared ray absorbing film is illustrated in Fig. 2. The obtained film had a maximum absorption wavelength of 788 nm, and a molar absorption coefficient in terms of compound No. 1 of ε = 1.1 × 10⁴, and this film showed fine absorption in the near-infrared ray region.

### [Example 2] Production of near-infrared ray absorbing synthetic resin composition and near-infrared ray absorbing material

A PMMA near-infrared ray absorbing film was obtained in a similar manner to Example 1, except that polymethyl methacrylate (PMMA) was used instead of polycarbonate, and that the thickness of the film was 30 µm.

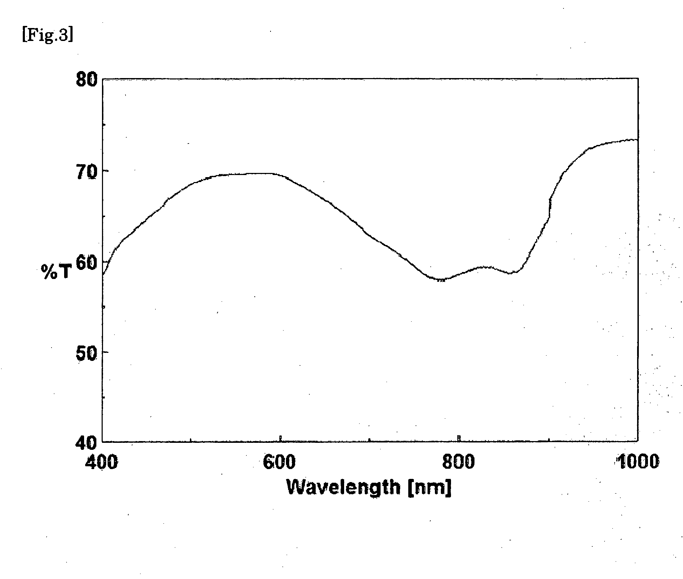

The absorption spectrum of the obtained PMMA near-infrared ray absorbing film is illustrated in Fig. 3. The obtained film had a maximum absorption wavelength of 779 nm, and a molar absorption coefficient in terms of compound No. 1 of ε = 1.3 × 10⁴, and this film showed fine absorption in the near-infrared ray region.

### [Evaluation Example 1]

The Haze values (transparency) of the near-infrared ray absorbing films obtained in Examples 1 and 2 were measured in reference to JISK7105. Measurements were also conducted on a polycarbonate film (Comparative Example 1) that was prepared in a similar manner to Example 1 except that compound No. 1 was not used, and a PMMA film (Comparative Example 2) that was prepared in a similar manner to Example 2 except that compound No. 1 was not used. The results of the measurements are illustrated in Table 1.

**[Table 1]**

| | Example 1 | Comparative Example 1 | Example 2 | Comparative Example 2 |
|---|---|---|---|---|
| Test Compound | Compound No.1 | None | Compound No.1 | None |
| Haze value(%) | 1.2 | 1.1 | 1.3 | 1.2 |

## Claims

1. A near-infrared ray absorbing synthetic resin composition containing a bipyrrolo isoquinoline compound represented by the following general formula (1) and a synthetic resin: 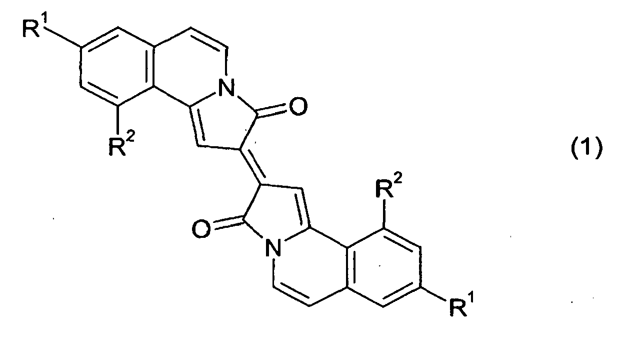 wherein R¹ and R² may be identical with or different from each other, and each represents a hydrogen atom, a halogen atom, an alkyl group having 1 to 20 carbon atoms optionally having substituents, an alkoxy group having 1 to 20 carbon atoms optionally having substituents, an aryl group having 6 to 20 carbon atoms optionally having substituents, an arylalkyl group having 7 to 20 carbon atoms optionally having substituents, or a cycloalkyl group having 5 to 12 carbon atoms optionally having substituents,
wherein the content of the bipyrrolo isoquinoline compound represented by the general formula (1) is 0.001 to 5 parts by mass with respect to 100 parts by mass of the synthetic resin.

2. The near-infrared ray absorbing synthetic resin composition according to claim 1, wherein the synthetic resin is a thermoplastic resin.

3. A near-infrared ray absorbing material formed by molding the near-infrared ray absorbing synthetic resin composition according to claim 1 or 2.

## Patentansprüche

1. Eine Nahinfrarotstrahlung absorbierende Kunstharzzusammensetzung enthaltend eine Bipyrroloisochinolin Verbindung, die durch die folgende allgemeine Formel (1) dargestellt wird, und ein Kunstharz: 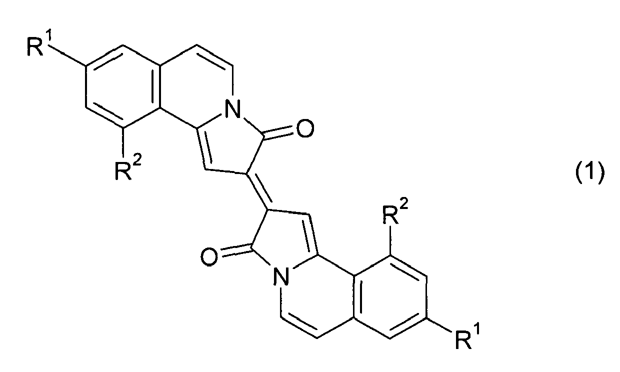 wobei R¹ und R² identisch oder voneinander verschieden sein können und jeweils ein Wasserstoffatom, ein Halogenatom, eine Alkylgruppe mit 1 bis 20 Kohlenstoffatomen, die optional Substituenten aufweist, eine Alkoxygruppe mit 1 bis 20 Kohlenstoffatomen, die optional Substituenten aufweist, eine Arylgruppe mit 6 bis 20 Kohlenstoffatomen, die optional Substituenten aufweist, eine Arylalkylgruppe mit 7 bis 20 Kohlenstoffatomen, die optional Substituenten aufweist, oder eine Cycloalkylgruppe mit 5 bis 12 Kohlenstoffatomen, die optional Substituenten aufweist, darstellen,
wobei der Gehalt der Bipyrroloisochinolin Verbindung, die durch die allgemeine Formel (1) dargestellt wird, 0,001 bis 5 Gewichtsteile beträgt, bezogen auf 100 Gewichtsteile des Kunstharzes.

2. Die Nahinfrarotstrahlung absorbierende Kunstharzzusammensetzung nach Anspruch 1, wobei das Kunstharz ein thermoplastisches Harz ist.

3. Ein Nahinfrarotstrahlung absorbierendes Material, das durch Formen der Nahinfrarotstrahlung absorbierenden Kunstharzzusammensetzung nach Anspruch 1 oder 2 gebildet wurde.

## Revendications

1. Composition de résine synthétique absorbant les rayons du proche infrarouge contenant un composé bipyrrolo isoquinoléine représenté par la formule générale suivante (1) et une résine synthétique: 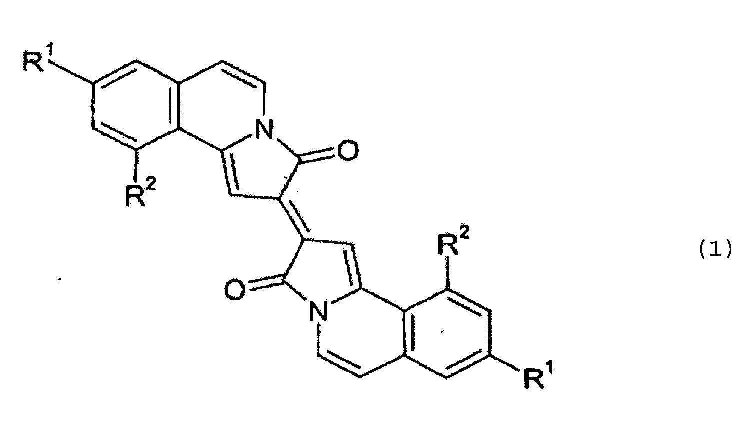 où R¹ et R² peuvent être identiques ou différents l'un de l'autre, et représentent chacun un atome d'hydrogène, un atome d'halogène, un groupe alkyle ayant de 1 à 20 atomes de carbone ayant éventuellement des substituants, un groupe alcoxy ayant de 1 à 20 atomes de carbone ayant éventuellement des substituants, un groupe aryle ayant de 6 à 20 atomes de carbone ayant éventuellement des substituants, un groupe arylalkyle ayant de 7 à 20 atomes de carbone ayant éventuellement des substituants, ou un groupe cycloalkyle ayant de 5 à 12 atomes de carbone ayant éventuellement des substituants,
dans laquelle la teneur du composé bipyrrolo isoquinoléine représenté par la formule générale (1) est de 0,001 à 5 parties en masse par rapport à 100 parties en masse de la résine synthétique.

2. Composition de résine synthétique absorbant les rayons du proche infrarouge selon la revendication 1, dans laquelle la résine synthétique est une résine thermoplastique.

3. Matériau absorbant les rayons du proche infrarouge formé par moulage de la composition de résine synthétique absorbant les rayons du proche infrarouge selon la revendication 1 ou 2.
